# EUROPÄISCHE PATENTSCHRIFT

(11) **EP 4 436 619 B1**
(45) Veröffentlichungstag und Bekanntmachung des Hinweises auf die Patenterteilung: **10.09.2025**
(21) Anmeldenummer: 22802917.9
(22) Anmeldetag: 12.10.2022
(51) Int. Cl.: A61L 9/12, B60H 3/00

(54) **BEDUFTUNGSVORRICHTUNG**
FRAGRANCING DEVICE
DISPOSITIF DIFFUSEUR DE PARFUM

(30) Priorität: 24.11.2021 DE 102021130779
(43) Veröffentlichungstag der Anmeldung: 02.10.2024
(73) Patentinhaber: Audi AG, 85045 Ingolstadt (DE)
(72) Erfinder: ROSS, Steffen, 85139 Wettstetten (DE); MOISSL, Martin, 85139 Wettstetten (DE)
(86) Internationale Anmeldenummer: PCT/EP2022/078328
(87) Internationale Veröffentlichungsnummer: WO 2023/094067

(56) Entgegenhaltungen:
- WO-A1-2021/116562
- DE-A1- 10 327 859
- DE-A1- 102013 221 351

## Beschreibung

Die Erfindung betrifft eine Beduftungsvorrichtung mit mindestens einem Duftstoffspeicher, insbesondere für ein Fahrzeug.

Beduftungsvorrichtungen für Fahrzeuge sind in zahlreichen Variationen bekannt. Die Beduftung der Fahrzeuginnenräume erfolgt in der Regel über Duftstoffspeicher, welche mit Duftstoffen befüllt sind. Diese Duftstoffspeicher werden in Intervallen geöffnet, um die Riechwahrnehmung zu optimieren. Der Öffnungsmechanismus wird in der Regel über elektromotorisch angetriebene Klappen umgesetzt. Hierbei können die Größe der Antriebe und Klappenmechanismen sehr viel Bauraum einnehmen. Zudem können durch den Aufbau von mehreren beweglichen Bauteilen Maßnahmen zur Geräuschminimierung erforderlich sein. Des Weiteren sind alle Bauteile Einzelkomponenten und können nur eine Aufgabe übernehmen. Dies kann zu hohen Kosten und Gewicht führen.

Aus der DE 10 2013 221 351 A1 ist eine Beduftungsvorrichtung bekannt, welche eine Duftmittelkartusche, einen Aktor und ein Klappenelement zur Öffnung und Schließung der Duftmittelkartusche umfasst. Die Duftmittelkartusche weist eine Öffnung auf, welche durch das durch den Aktor bewegbare Klappenelement öffenbar und verschließbar ist. Der Aktor ist aus einer Formgedächtnislegierung und/oder aus einem Bimetall gebildet und einteilig mit dem Klappenelement ausgeführt.

Aus der WO 2021/116562 A1 ist eine Vorrichtung zur Abgabe eines Duftstoffes bekannt, welche zum Einbau in einen Fahrzeuginnenraum bestimmt ist und einen Behälter zum Aufbewahren eines Duftstoffes umfasst, welcher durch eine Zerstäubungseinrichtung in den Fahrgastraum diffundiert werden kann. Zudem umfasst die Vorrichtung einen mit dem Behälter verbundenen Kanal und ein Verschlusselement, welches in der Lage ist, das Öffnen und Schließen des Kanals zu verwalten. Das Verschlusselement umfasst mindestens ein elektroaktives Element, welches eine den Kanal verschließende Position einnimmt, wenn das mindestens eine elektroaktive Element einer ersten Spannung ausgesetzt ist, und eine den Kanal öffnende Position einnimmt, wenn das mindestens eine elektroaktive Element mit einer von der ersten Spannung verschiedenen zweiten Spannung beaufschlagt wird, welche insbesondere höher als die erste Spannung ist.

Der Erfindung liegt die Aufgabe zu Grunde, eine Beduftungsvorrichtung mit mindestens einem Duftstoffspeicher bereitzustellen, welche ein geräuscharmes Öffnen und Schließen des mindestens einen Duftstoffspeichers bei geringem Bauraumbedarf und geringem Gewicht ermöglicht.

Diese Aufgabe wird durch eine Beduftungsvorrichtung mit den Merkmalen des Patentanspruchs 1 gelöst. Vorteilhafte Ausgestaltungen mit zweckmäßigen Weiterbildungen der Erfindung sind in den abhängigen Patentansprüchen angegeben.

Um eine Beduftungsvorrichtung mit mindestens einem Duftstoffspeicher bereitzustellen, welche ein geräuscharmes Öffnen und Schließen des mindestens einen Duftstoffspeichers bei geringem Bauraumbedarf und geringem Gewicht ermöglicht, ist eine Austrittsöffnung des mindestens einen Duftstoffspeichers über eine Verschlussvorrichtung verschließbar, welche als flexibler Leiterplattenaktor ausgebildet ist. Hierbei ist der flexible Leiterplattenaktor in mindestens einem Bereich zweilagig ausgebildet, so dass ein Abdeckabschnitt des Leiterplattenaktors die Austrittsöffnung im unbestromten Zustand abdichtet und die Austrittsöffnung im bestromten Zustand freigibt. Der flexible Leiterplattenaktor umfasst mindestens eine flexible Leiterplatte mit einer bestrombaren ersten Spulenstruktur und mit einer bestrombaren zweiten Spulenstruktur. Alternativ umfasst der flexible Leiterplattenaktor mindestens eine flexible Leiterplatte mit einer bestrombaren ersten Spulenstruktur und mit einem Permanentmagneten.

Vorzugsweise können die beiden bestrombaren Spulenstrukturen elektrisch in Reihe geschaltet sein. Herbei können im montierten Zustand des flexiblen Leiterplattenaktors die erste Spulenstruktur und der Abdeckabschnitt in einer ersten Lage der mindestens einen flexiblen Leiterplatte angeordnet sein, welche der Austrittsöffnung des Duftstoffspeichers zugewandt ist. Die zweite Spulenstruktur kann in einer zweiten Lage der mindestens einen flexiblen Leiterplatte angeordnet sein, welche von der Austrittsöffnung des Duftstoffspeichers abgewandt ist. Zudem können die beiden Lagen der mindestens einen flexiblen Leiterplatte vorzugsweise so zueinander ausgerichtet sein, dass die beiden Spulenstrukturen übereinander angeordnet sind. Hierbei können die Spulenstrukturen beispielsweise in zwei separaten flexiblen Leiterplatten angeordnet sein, welche jeweils eine der beiden Lagen ausbilden. Die beiden flexiblen Leiterplatte können mit einem vorgegebenen Abstand zueinander übereinander angeordnet sein.

Bei der alternativen Ausgestaltung der Beduftungsvorrichtung können im montierten Zustand des flexiblen Leiterplattenaktors die erste Spulenstruktur und der Abdeckabschnitt in einer ersten Lage der mindestens einen flexiblen Leiterplatte angeordnet sein, welche der Austrittsöffnung des Duftstoffspeichers zugewandt ist. Der Permanentmagnet kann in einer zweiten Lage der mindestens einen flexiblen Leiterplatte angeordnet sein, welche von der Austrittsöffnung des Duftstoffspeichers abgewandt ist. Vorzugsweise können die beiden Lagen der mindestens einen flexiblen Leiterplatte so zueinander ausgerichtet sein, dass die erste Spulenstruktur und der Permanentmagnet übereinander angeordnet sind. Hierbei kann die erste Spulenstruktur und der Permanentmagnet beispielsweise in zwei separaten flexiblen Leiterplatten angeordnet sein, welche jeweils eine der beiden Lagen ausbilden. Die beiden flexiblen Leiterplatte können mit einem vorgegebenen Abstand zueinander übereinander angeordnet sein.

In weiterer vorteilhafter Ausgestaltung der Beduftungsvorrichtung kann mindestens ein Federelement zumindest den Abdeckabschnitt der ersten Lage der mindestens einen flexiblen Leiterplatte mit der ersten Spulenstruktur dichtend gegen einen Rand der Austrittsöffnung drücken, so dass die beiden Lagen der mindestens einen flexiblen Leiterplatte im unbestromten Zustand einen vorgegebenen Abstand zueinander aufweisen. Bei der Ausführung des flexiblen Leiterplattenaktors mit zwei Spulenstrukturen kann eine durch die Bestromung der beiden Spulenstrukturen erzeugte Magnetkraft die der Austrittsöffnung zugewandte erste Lage mit der ersten Spulenstruktur und dem Abdeckabschnitt vom Rand der Austrittsöffnung in Richtung der zweiten Spulenstruktur in der zweiten Lage der flexiblen Leiterplatte bewegen, so dass der Abdeckabschnitt der ersten Lage die Austrittsöffnung freigibt. Bei der Ausführung des flexiblen Leiterplattenaktors mit einer ersten Spulenstruktur und einem Permanentmagneten kann eine durch Bestromung der ersten Spulenstruktur und durch den Permanentmagneten erzeugte Magnetkraft die der Austrittsöffnung zugewandte erste Lage der mindestens einen flexiblen Leiterplatte mit der ersten Spulenstruktur und dem Abdeckabschnitt gegen die Kraft des mindestens einen Federelements vom Rand der Austrittsöffnung in Richtung Permanentmagnet in der zweiten Lage der mindestens einen flexiblen Leiterplatte bewegen, so dass der Abdeckabschnitt der ersten Lage die Austrittsöffnung freigibt.

Durch die Ausführung der Verschlussvorrichtung auf Basis von gedruckten Schaltungen auf einer flexiblen Leiterplatte, ist eine geräuscharme Bewegung der ersten Lage der flexiblen Leiterplatte bei geringem Bauraumbedarf möglich. Zudem übernimmt die erste Lage der flexiblen Leiterplatte zugleich die Abdichtung der Austrittsöffnung. Die flexible Leiterplatte mit den bedruckten Spulenstrukturen kann als Massenprodukt einfach und kostengünstig bei geringem Gewicht hergestellt werden.

In weiterer vorteilhafter Ausgestaltung der Beduftungsvorrichtung kann die erste Spulenstruktur und die zweite Spulenstruktur bzw. die erste Spulenstruktur und der Permanentmagnet in einer gemeinsamen flexiblen Leiterplatte angeordnet sein, welche zwischen den beiden Spulenstrukturen bzw. zwischen der ersten Spulenstruktur und dem Permanentmagneten einen ersten Wellenschlag aufweist, welcher die gemeinsame flexible Leiterplatte umlenkt und ein erstes Federelement ausbildet. Dadurch erfolgt die Rückstellung der ersten Lage über die federnden Eigenschaften der flexiblen Leiterplatte. Dadurch kann die Anzahl der Komponenten zur Umsetzung des flexiblen Leiterplattenaktors weiter reduziert werden. Hierbei kann der erste Wellenschlag so ausgebildet werden, dass die beiden Lagen der gemeinsamen flexiblen Leiterplatte im unbestromten Zustand einen vorgegebenen Abstand zueinander aufweisen.

**In** weiterer vorteilhafter Ausgestaltung der Beduftungsvorrichtung kann ein Endabschnitt der gemeinsamen flexiblen Leiterplatte einen zweiten Wellenschlag aufweisen, welcher die gemeinsame flexible Leiterplatte nochmals umlenken und ein zweites Federelement ausbilden kann, so dass der der Austrittsöffnung zugewandte Abdeckabschnitt der ersten Lage der gemeinsamen flexiblen Leiterplatte gleichmäßig gegen den Rand der Austrittsöffnung gedrückt werden kann. Der Endabschnitt der gemeinsamen flexiblen Leiterplatte kann nach dem zweiten Wellenschlag an der ersten Lage befestigt werden.

In weiterer vorteilhafter Ausgestaltung der Beduftungsvorrichtung kann die flexible Leiterplatte im Bereich des ersten Wellenschlags und im Bereich des zweiten Wellenschlags jeweils eine Aussparung aufweisen, so dass das erste Federelement und das zweite Federelement jeweils zwei Federschenkel aufweisen können. Durch Abmessungen und Form der Aussparung können die erzeugten Federkräfte der beiden Federelemente vorgegeben und an verschiedenen Ausführungsformen angepasst werden.

In weiterer vorteilhafter Ausgestaltung der Beduftungsvorrichtung kann eine der Austrittsöffnung zugewandte Oberfläche des Abdeckabschnitts der ersten Lage der mindestens einen flexiblen Leiterplatte als Dichtfläche ausgebildet sein. Hierzu kann eine entsprechende Beschichtung bzw. ein entsprechendes Dichtmaterial auf den Abdeckabschnitt der flexiblen Leiterplatte aufgebracht werden.

Ausführungsbeispiele der Erfindung sind in den Zeichnungen dargestellt und werden in der nachfolgenden Beschreibung näher erläutert. In den Zeichnungen bezeichnen gleiche Bezugszeichen Komponenten bzw. Elemente, die gleiche bzw. analoge Funktionen ausführen. Hierbei zeigen:
- Fig. 1: eine schematische und ausschnittsweise Darstellung eines ersten Ausführungsbeispiels einer erfindungsgemäßen Beduftungsvorrichtung mit mindestens einem Duftstoffspeicher in einer Offenstellung;
- Fig. 2: eine schematische und ausschnittsweise Darstellung der erfindungsgemäßen Beduftungsvorrichtung in einer Geschlossenstellung;
- Fig. 3: eine schematische Darstellung eines ersten Ausführungsbeispiels einer flexiblen Leiterplatte für eine Verschlussvorrichtung der erfindungsgemäßen Beduftungsvorrichtung aus Fig. 1 und 2 vor dem Zusammenbau;
- Fig. 4: eine schematische und perspektivische Darstellung der zusammengebauten Verschlussvorrichtung aus Fig. 3;
- Fig. 5: eine schematische Darstellung eines zweiten Ausführungsbeispiels einer flexiblen Leiterplatte für eine Verschlussvorrichtung der erfindungsgemäßen Beduftungsvorrichtung aus Fig. 1 und 2 vor dem Zusammenbau; und
- Fig. 6: eine schematische und perspektivische Darstellung der zusammengebauten Verschlussvorrichtung aus Fig. 5;

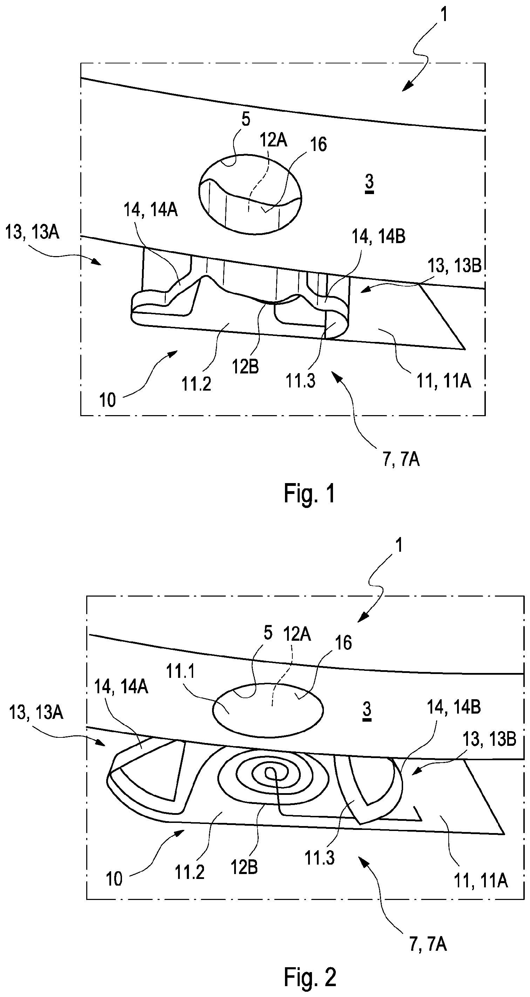

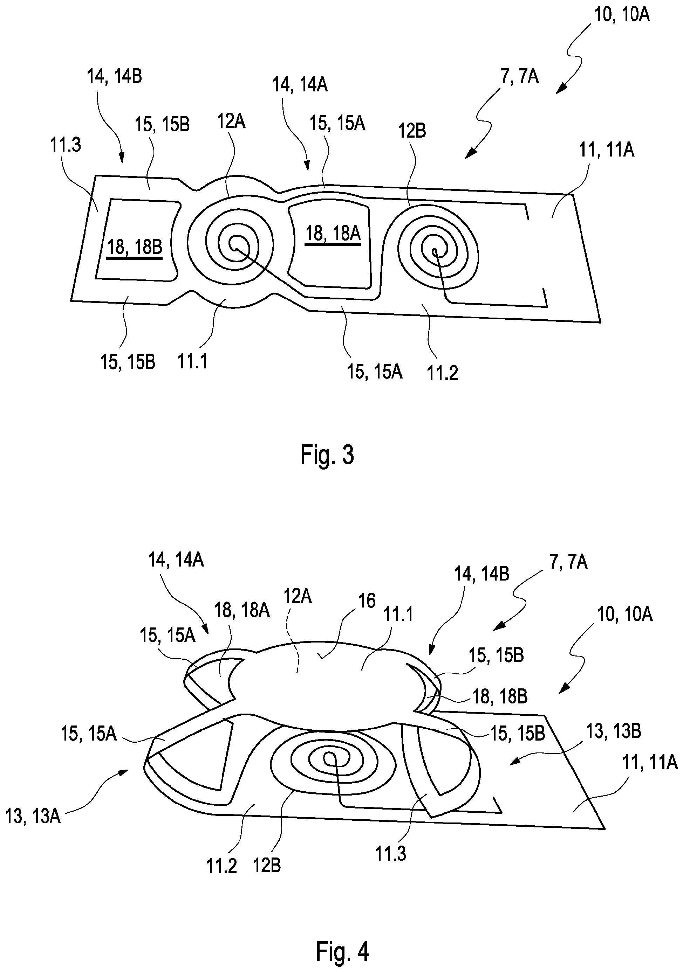

Wie aus Fig. 1 bis 6 ersichtlich ist, umfasst das dargestellte erste Ausführungsbeispiel einer erfindungsgemäßen Beduftungsvorrichtung 1 mindestens einen ausschnittsweise dargestellten Duftstoffspeicher 3. Der mindestens eine Duftstoffspeicher 3 weist eine Austrittsöffnung 5 auf, welche über eine Verschlussvorrichtung 7, 7A, 7B verschließbar ist. Hierbei ist die Verschlussvorrichtung 7, 7A, 7B als flexibler Leiterplattenaktor 10, 10A, 10B ausgebildet, wobei der flexible Leiterplattenaktor 10, 10A, 10B in mindestens einem Bereich zweilagig ausgebildet ist, so dass ein Abdeckabschnitt 16 des flexiblen Leiterplattenaktors 10, 10A, 10B die Austrittsöffnung 5 im unbestromten Zustand abdichtet und die Austrittsöffnung 5 im bestromten Zustand freigibt.

Wie aus Fig. 1 bis 6 weiter ersichtlich ist, umfasst der flexible Leiterplattenaktor 10A, 10B in den dargestellten Ausführungsbeispielen jeweils mindestens eine flexible Leiterplatte 11, 11A, 11B mit einer bestrombaren ersten Spulenstruktur 12A und mit einer bestrombaren zweiten Spulenstruktur 12B. Hierbei sind im montierten Zustand des flexiblen Leiterplattenaktors 10, 10A, 10B die erste Spulenstruktur 12A und der Abdeckabschnitt 16 in einer ersten Lage 11.1 der mindestens einen flexiblen Leiterplatte 11, 11A, 11B angeordnet, welche der Austrittsöffnung 5 des Duftstoffspeichers 3 zugewandt ist. Die zweite Spulenstruktur 12B ist in einer zweiten Lage 11.2 der mindestens einen flexiblen Leiterplatte 11, 11A, 11B angeordnet, welche von der Austrittsöffnung 5 des Duftstoffspeichers 3 abgewandt ist.

Wie aus Fig. 1, 2, 4 und 6 weiter ersichtlich ist, sind die beiden Lagen 11.1, 11.2 der mindestens einen flexiblen Leiterplatte 11, 11A, 11B so zueinander ausgerichtet, dass die beiden Spulenstrukturen 12A, 12B übereinander angeordnet sind. Hierbei drückt mindestens ein Federelement 14 zumindest den Abdeckabschnitt 16 der ersten Lage 11.1 der mindestens einen flexiblen Leiterplatte 11, 11A, 11B mit der ersten Spulenstruktur 12A dichtend gegen einen Rand der Austrittsöffnung 5, so dass die beiden Lagen 11.1, 11.2 der mindestens einen flexiblen Leiterplatte 11, 11A, 11B im unbestromten Zustand einen vorgegebenen Abstand zueinander aufweisen. Eine durch die Bestromung der beiden Spulenstrukturen 12A, 12B erzeugte Magnetkraft bewegt die der Austrittsöffnung 5 zugewandte erste Lage 11.1 der mindestens einen flexiblen Leiterplatte 11, 11A, 11B mit der ersten Spulenstruktur 12A und dem Abdeckabschnitt 16 gegen die Kraft des mindestens einen Federelements 14 vom Rand der Austrittsöffnung 5 in Richtung der zweiten Spulenstruktur 12B in der zweiten Lage 11.2 der mindestens einen flexiblen Leiterplatte 11, 11A, 11B, so dass der Abdeckabschnitt 16 die Austrittsöffnung 5 im bestromten Zustand freigibt.

Zur Verbesserung der Abdichteigenschaft ist eine der Austrittsöffnung 5 zugewandte Oberfläche des Abdeckabschnitts 16 der ersten Lage 11.1 der mindestens einen flexiblen Leiterplatte 11, 11A, 11B als Dichtfläche ausgebildet. Hierzu ist in den beschriebenen Ausführungsbeispielen ein geeignetes Dichtungsmaterial auf den Abdeckabschnitt 16 der ersten Lage 11.1 aufgebracht. **In** den dargestellten Ausführungsbeispielen ist der als Dichtfläche ausgebildete Abdeckabschnitt 16 im Bereich der ersten Spulenstruktur 12A an der ersten Lage 11.1 der mindestens einen Leiterplatte 11, 11A, 11B angeordnet. Bei alternativen nicht dargestellten Ausführungsbeispielen ist der Abdeckabschnitt 16 benachbart, d.h. vor oder nach bzw. neben der ersten Spulenstruktur 12A an der ersten Lage 11.1 der mindestens einen Leiterplatte 11, 11A, 11B angeordnet.

Wie aus Fig. 1 bis 6 weiter ersichtlich ist, sind die erste Spulenstruktur 12A und die zweite Spulenstruktur 12B in einer gemeinsamen flexiblen Leiterplatte 11, 11A, 11B angeordnet und elektrisch in Reihe geschaltet. Die gemeinsame flexible Leiterplatte 11, 11A, 11B weist zwischen den beiden Spulenstrukturen 12A, 12B einen ersten Wellenschlag 13A auf, welcher die gemeinsame flexible Leiterplatte 11, 11A, 11B umlenkt und ein erstes Federelement 14A ausbildet. Zudem weist ein Endabschnitt 11.3 der flexiblen Leiterplatte 11, 11A, 11B in den dargestellten Ausführungsbeispielen einen zweiten Wellenschlag 13B auf, welcher die flexible Leiterplatte 11, 11A, 11B umlenkt und ein zweites Federelement 14B ausbildet, so dass der der Austrittsöffnung 5 zugewandte Abdeckabschnitt 16 der ersten Lage 11.1 der flexiblen Leiterplatte 11, 11A, 11B gleichmäßig gegen den Rand der Austrittsöffnung 5 drückbar ist.

Bei einem nicht dargestellten Ausführungsbeispiel des flexiblen Leiterplattenaktors 10 sind die erste Spulenstruktur 12A und die zweite Spulenstruktur 12B jeweils in einer flexiblen Leiterplatte 11 angeordnet. Hierbei sind die beiden flexiblen Leiterplatten 11 übereinander angeordnet und über mindestens ein Federelement 14 miteinander gekoppelt.

Wie aus Fig. 1 bis 4 weiter ersichtlich ist, weist die flexible Leiterplatte 11A im dargestellten ersten Ausführungsbeispiel des flexiblen Leiterplattenaktors 10A im Bereich des ersten Wellenschlags 13A und im Bereich des zweiten Wellenschlags 13B jeweils eine Aussparung 18, 18A, 18B auf, so dass das erste Federelement 14A und das zweite Federelement 14B jeweils zwei Federschenkel 15, 15A, 15B aufweisen. Das bedeutet, dass in den zwischen der ersten Spulenstruktur 12A und der zweiten Spulenstruktur 12B angeordneten Bereich der flexiblen Leiterplatte 11A eine erste Aussparung 18A eingebracht ist und das erste Federelement 14A zwei erste Federschenkel 15A aufweist. Des Weiteren ist hinter der ersten Spulenstruktur 12A in den Endbereich 11.3 der flexiblen Leiterplatte 11A eine zweite Aussparung 18B eingebracht, so dass das zweite Federelement 14B zwei zweite Federschenkel 15B aufweist.

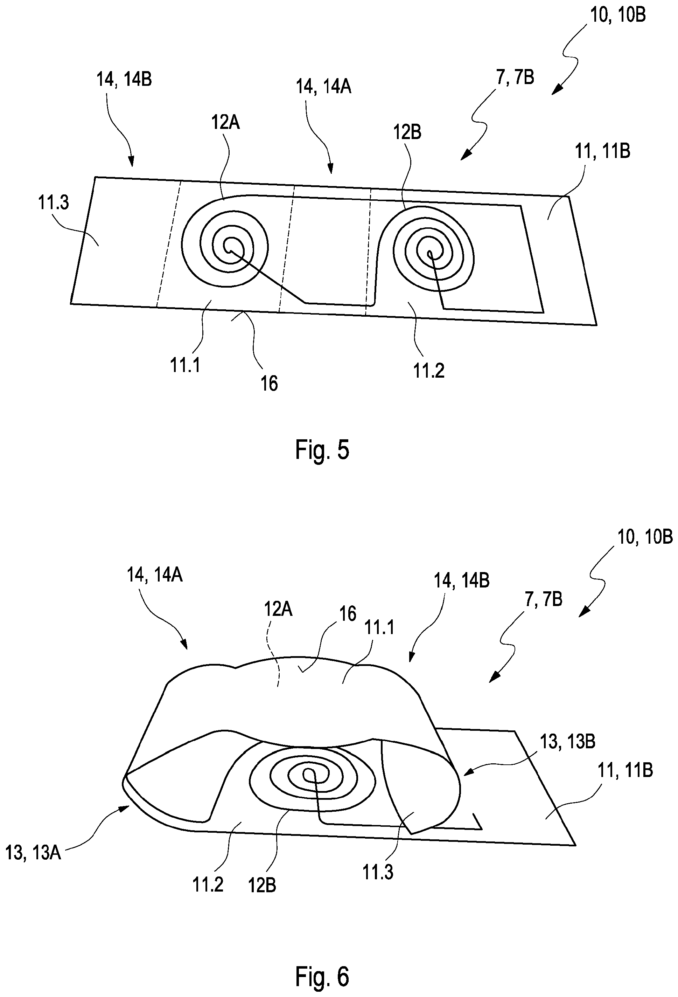

Wie aus Fig. 5 und 6 weiter ersichtlich ist, weist die flexible Leiterplatte 11B im dargestellten zweiten Ausführungsbeispiel des flexiblen Leiterplattenaktors 10B im Unterschied zum ersten Ausführungsbeispiel des flexiblen Leiterplattenaktors 10A keine Aussparungen 18, 18A, 18B im Bereich des ersten Wellenschlags 13A und im Bereich des zweiten Wellenschlags 13B auf. Daher bildet im dargestellten zweiten Ausführungsbeispiel des flexiblen Leiterplattenaktors der gesamte erste Wellenschlag 13A das erste Federelement 14A aus, und der gesamte zweite Wellenschlag 13B bildet das zweite Federelement 14B aus.

Bei einem nicht dargestellten zweiten Ausführungsbeispiel der erfindungsgemäßen Beduftungsvorrichtung 1 umfasst der flexible Leiterplattenaktor 10 mindestens eine flexible Leiterplatte 11 mit einer bestrombaren ersten Spulenstruktur 12A und mit einem Permanentmagneten. Bei dem alternativen Ausführungsbeispiel ist im montierten Zustand des flexiblen Leiterplattenaktors 10 die erste Spulenstruktur 12A und der Abdeckabschnitt 16 in einer ersten Lage 11.1 der mindestens einen flexiblen Leiterplatte 11 angeordnet, welche der Austrittsöffnung 5 des Duftstoffspeichers 3 zugewandt ist. Im Unterschied zu dem in Verbindung mit Fig. 1 bis 6 beschriebenen ersten Ausführungsbeispiel der Beduftungsvorrichtung 1 ist anstelle der zweiten Spulenstruktur 12B der Permanentmagnet in der zweiten Lage 11.2 der mindestens einen flexiblen Leiterplatte 11 angeordnet, welche von der Austrittsöffnung 5 des Duftstoffspeichers 3 abgewandt ist. Analog zu dem oben beschriebenen Ausführungsbeispiel sind die beiden Lagen 11.1, 11.2 der mindestens einen flexiblen Leiterplatte 11 so zueinander ausgerichtet, dass die erste Spulenstruktur 12A und der Permanentmagnet übereinander angeordnet sind. Hierbei bewegt eine durch Bestromung der ersten Spulenstruktur 12A und durch den Permanentmagneten erzeugte Magnetkraft die der Austrittsöffnung 5 zugewandte erste Lage 11.1 der mindestens einen flexiblen Leiterplatte 11 mit der ersten Spulenstruktur 12A und dem Abdeckabschnitt 16 gegen die Kraft des mindestens einen Federelements 14 vom Rand der Austrittsöffnung 5 in Richtung Permanentmagnet in der zweiten Lage 11.2 der mindestens einen flexiblen Leiterplatte 11, so dass der Abdeckabschnitt 16 der ersten Lage 11.1 die Austrittsöffnung 5 freigibt.

Analog zum ersten Ausführungsbeispiel der Beduftungsvorrichtung 1 sind auch bei dem nicht dargestellten zweiten Ausführungsbeispiel der Beduftungsvorrichtung 1 die erste Spulenstruktur 12A und der Permanentmagnet in einer gemeinsamen flexiblen Leiterplatte 11 angeordnet, welche zwischen der ersten Spulenstruktur 12A und dem Permanentmagneten einen ersten Wellenschlag 13A aufweist, welcher die gemeinsame flexible Leiterplatte 11 umlenkt und ein erstes Federelement 14A ausbildet. Zudem weist ein Endabschnitt 11.3 der flexiblen Leiterplatte 11 in dem dargestellten Ausführungsbeispiel einen zweiten Wellenschlag 13B auf, welcher die flexible Leiterplatte 11 umlenkt und ein zweites Federelement 14B ausbildet, so dass der der Austrittsöffnung 5 zugewandte Abdeckabschnitt 16 der ersten Lage 11.1 der flexiblen Leiterplatte 11 gleichmäßig gegen den Rand der Austrittsöffnung 5 drückbar ist. Alternativ kann die erste Spulenstruktur 12A und die zweite Spulenstruktur 12B des flexiblen Leiterplattenaktors 10 jeweils in einer flexiblen Leiterplatte 11 angeordnet sein. Hierbei sind die beiden flexiblen Leiterplatten 11 übereinander angeordnet und über mindestens ein Federelement 14 miteinander gekoppelt.

### BEZUGSZEICHENLISTE

- 1: Beduftungsvorrichtung
- 3: Duftstoffspeicher
- 5: Austrittsöffnung
- 7, 7A, 7B: Verschlussvorrichtung
- 10, 10A, 10B: flexibler Leiterplattenaktor
- 11,11A, 11B: flexible Leiterplatte
- 11.1, 11.2: Leiterplattenlage
- 11.3: Endabschnitt
- 12A, 12B: Spulenstruktur
- 13A, 13B: Wellenschlag
- 14, 14A, 14B,: Federelement
- 15, 15A, 15B: Federschenkel
- 16: Abdeckabschnitt
- 18, 18A, 18B: Aussparung

## Patentansprüche

1. Beduftungsvorrichtung (1) mit mindestens einem Duftstoffspeicher (3), wobei der mindestens eine Duftstoffspeicher (3) eine Austrittsöffnung (5) aufweist, welche über eine Verschlussvorrichtung (7) verschließbar ist, wobei die Verschlussvorrichtung (7) als flexibler Leiterplattenaktor (10) ausgebildet ist, wobei der flexible Leiterplattenaktor (10) in mindestens einem Bereich zweilagig ausgebildet ist, so dass ein Abdeckabschnitt (16) des Leiterplattenaktors (10) die Austrittsöffnung (5) im unbestromten Zustand abdichtet und im bestromten Zustand freigibt,
**gekennzeichnet dadurch, dass** der flexible Leiterplattenaktor (10) mindestens eine flexible Leiterplatte (11) mit einer bestrombaren ersten Spulenstruktur (12A) und mit einer bestrombaren zweiten Spulenstruktur (12B) oder mindestens eine flexible Leiterplatte (11) mit einer bestrombaren ersten Spulenstruktur (12A) und mit einem Permanentmagneten umfasst.

2. Beduftungsvorrichtung (1) nach Anspruch 1,
**dadurch gekennzeichnet,**
**dass** im montierten Zustand des flexiblen Leiterplattenaktors (10) die erste Spulenstruktur (12A) und der Abdeckabschnitt (16) in einer ersten Lage (11.1) der mindestens einen flexiblen Leiterplatte (11) angeordnet sind, welche der Austrittsöffnung (5) des Duftstoffspeichers (3) zugewandt ist, und die zweite Spulenstruktur (12B) in einer zweiten Lage (11.2) der mindestens einen flexiblen Leiterplatte (11) angeordnet ist, welche von der Austrittsöffnung (5) des Duftstoffspeichers (3) abgewandt ist.

3. Beduftungsvorrichtung (1) nach Anspruch 2,
**dadurch gekennzeichnet,**
**dass** die beiden Lagen (11.1, 11.2) der mindestens einen flexiblen Leiterplatte (11) so zueinander ausgerichtet sind, dass die beiden Spulenstrukturen (12A, 12B) übereinander angeordnet sind.

4. Beduftungsvorrichtung (1) nach Anspruch 1,
**dadurch gekennzeichnet,**
**dass** im montierten Zustand des flexiblen Leiterplattenaktors (10) die erste Spulenstruktur (12A) und der Abdeckabschnitt (16) in einer ersten Lage (11.1) der mindestens einen flexiblen Leiterplatte (11) angeordnet sind, welche der Austrittsöffnung (5) des Duftstoffspeichers (3) zugewandt ist, und der Permanentmagnet in einer zweiten Lage (11.2) der mindestens einen flexiblen Leiterplatte (11) angeordnet ist, welche von der Austrittsöffnung (5) des Duftstoffspeichers (3) abgewandt ist.

5. Beduftungsvorrichtung (1) nach Anspruch 4,
**dadurch gekennzeichnet,**
**dass** die beiden Lagen (11.1, 11.2) der mindestens einen flexiblen Leiterplatte (11) so zueinander ausgerichtet sind, dass die erste Spulenstruktur (12A) und der Permanentmagnet übereinander angeordnet sind.

6. Beduftungsvorrichtung (1) nach einem der Ansprüche 2 bis 5,
**dadurch gekennzeichnet,**
**dass** mindestens ein Federelement (14) zumindest den Abdeckabschnitt (16) der ersten Lage (11.1) der mindestens einen flexiblen Leiterplatte (11) mit der ersten Spulenstruktur (12A) dichtend gegen einen Rand der Austrittsöffnung (5) drückt, so dass die beiden Lagen (11.1, 11.2) der mindestens einen flexiblen Leiterplatte (11) im unbestromten Zustand einen vorgegebenen Abstand zueinander aufweisen.

7. Beduftungsvorrichtung (1) nach Anspruch 2 und 6,
**dadurch gekennzeichnet,**
**dass** eine durch die Bestromung der beiden Spulenstrukturen (12A, 12B) erzeugte Magnetkraft die der Austrittsöffnung (5) zugewandte erste Lage (11.1) der mindestens einen flexiblen Leiterplatte (11) mit der ersten Spulenstruktur (12A) und dem Abdeckabschnitt (16) gegen die Kraft des mindestens einen Federelements (14) vom Rand der Austrittsöffnung (5) in Richtung der zweiten Spulenstruktur (12B) in der zweiten Lage (11.2) der mindestens einen flexiblen Leiterplatte (11) bewegt, so dass der Abdeckabschnitt (16) der ersten Lage (11.1) die Austrittsöffnung (5) freigibt.

8. Beduftungsvorrichtung (1) nach Anspruch 6 oder 7,
**dadurch gekennzeichnet,**
**dass** die erste Spulenstruktur (12A) und die zweite Spulenstruktur (12B) in einer gemeinsamen flexiblen Leiterplatte (11) angeordnet sind, welche zwischen den beiden Spulenstrukturen (12A, 12B) einen ersten Wellenschlag (13A) aufweist, welcher die gemeinsame flexible Leiterplatte (11) umlenkt und ein erstes Federelement (14A) ausbildet.

9. Beduftungsvorrichtung (1) nach Anspruch 4 und 6,
**dadurch gekennzeichnet,**
**dass** eine durch Bestromung der ersten Spulenstruktur (12A) und durch den Permanentmagneten erzeugte Magnetkraft die der Austrittsöffnung (5) zugewandte erste Lage (11.1) der mindestens einen flexiblen Leiterplatte (11) mit der ersten Spulenstruktur (12A) und dem Abdeckabschnitt gegen die Kraft des mindestens einen Federelements (14) vom Rand der Austrittsöffnung (5) in Richtung Permanentmagnet in der zweiten Lage (11.2) der mindestens einen flexiblen Leiterplatte (11) bewegt, so dass der Abdeckabschnitt (16) der ersten Lage (11.1) die Austrittsöffnung (5) freigibt.

10. Beduftungsvorrichtung (1) nach Anspruch 6 oder 9,
**dadurch gekennzeichnet,**
**dass** die erste Spulenstruktur (12A) und der Permanentmagnet in einer gemeinsamen flexiblen Leiterplatte (11) angeordnet sind, welche zwischen der ersten Spulenstruktur (12A) und dem Permanentmagneten einen ersten Wellenschlag (13A) aufweist, welcher die gemeinsame flexible Leiterplatte (11) umlenkt und ein erstes Federelement (14A) ausbildet.

11. Beduftungsvorrichtung (1) nach Anspruch 8 oder 10,
**dadurch gekennzeichnet,**
**dass** ein Endabschnitt (11.3) der gemeinsamen flexiblen Leiterplatte (11) einen zweiten Wellenschlag (13B) aufweist, welcher die gemeinsame flexible Leiterplatte (11) umlenkt und ein zweites Federelement (14B) ausbildet, so dass der der Austrittsöffnung (5) zugewandte Abdeckabschnitt (16) der ersten Lage (11.1) der gemeinsamen flexiblen Leiterplatte (11) gleichmäßig gegen den Rand der Austrittsöffnung (5) drückbar ist.

12. Beduftungsvorrichtung (1) nach Anspruch 11,
**dadurch gekennzeichnet,**
**dass** die gemeinsame flexible Leiterplatte (11) im Bereich des ersten Wellenschlags (13A) und im Bereich des zweiten Wellenschlags (13B) jeweils eine Aussparung (18) aufweist, so dass das erste Federelement (14A) und das zweite Federelement (14B) jeweils zwei Federschenkel (15) aufweisen.

13. Beduftungsvorrichtung (1) nach einem der Ansprüche 2 bis 12,
**dadurch gekennzeichnet,**
**dass** eine der Austrittsöffnung (5) zugewandte Oberfläche des Abdeckabschnitts (16) der ersten Lage (11.1) der mindestens einen flexiblen Leiterplatte (11) als Dichtfläche ausgebildet ist.

## Claims

1. Fragrancing device (1) with at least one fragrance reservoir (3), wherein the at least one fragrance reservoir (3) has an outlet opening (5), which can be closed by a closing device (7), wherein the closing device (7) is designed as a flexible printed circuit board actuator (10), wherein the flexible printed circuit board actuator (10) is formed in two layers in at least one region, such that a cover section (16) of the printed circuit board actuator (10) seals the outlet opening (5) in the deenergized state and opens it in the energized state, **characterized in that** the flexible printed circuit board actuator (10) comprises at least one flexible printed circuit board (11) with a first coil structure (12A) that can be energized and with a second coil structure (12B) that can be energized or at least one flexible printed circuit board (11) with a first coil structure (12A) that can be energized and with a permanent magnet.

2. Fragrancing device (1) according to claim 1,
**characterized in that**
in the mounted state of the flexible printed circuit board actuator (10), the first coil structure (12A) and the cover section (16) are arranged in a first layer (11.1) of the at least one flexible printed circuit board (11), which faces the outlet opening (5) of the fragrance reservoir (3), and the second coil structure (12B) is arranged in a second layer (11.2) of the at least one flexible printed circuit board (11), which faces away from the outlet opening (5) of the fragrance reservoir (3).

3. Fragrancing device (1) according to claim 2,
**characterized in that**
the two layers (11.1, 11.2) of the at least one flexible printed circuit board (11) are aligned relative to one another such that the two coil structures (12A, 12B) are arranged above one another.

4. Fragrancing device (1) according to claim 1,
**characterized in that**
in the mounted state of the flexible printed circuit board actuator (10), the first coil structure (12A) and the cover section (16) are arranged in a first layer (11.1) of the at least one flexible printed circuit board (11), which faces the outlet opening (5) of the fragrance reservoir (3), and the permanent magnet is arranged in a second layer (11.2) of the at least one flexible printed circuit board (11), which faces away from the outlet opening (5) of the fragrance reservoir (3).

5. Fragrancing device (1) according to claim 4,
**characterized in that**
the two layers (11.1, 11.2) of the at least one flexible printed circuit board (11) are aligned relative to one another such that the first coil structure (12A) and the permanent magnet are arranged above one another.

6. Fragrancing device (1) according to any one of claims 2 to 5,
**characterized in that**
at least one spring element (14) presses at least the cover section (16) of the first layer (11.1) of the at least one flexible printed circuit board (11) with the first coil structure (12A) in a sealing manner against an edge of the outlet opening (5), such that the two layers (11.1, 11.2) of the at least one flexible printed circuit board (11) have a predetermined distance from one another in the deenergized state.

7. Fragrancing device (1) according to claim 2 and 6,
**characterized in that**
a magnetic force generated by the energization of the two coil structures (12A, 12B), which moves the first layer (11.1) of the at least one flexible printed circuit board (11) with the first coil structure (12A) facing the outlet opening (5) and the cover section (16) against the force of the at least one spring element (14) from the edge of the outlet opening (5) in the direction of the second coil structure (12B) in the second layer (11.2) of the at least one flexible printed circuit board (11), such that the cover section (16) of the first layer (11.1) opens the outlet opening (5).

8. Fragrancing device (1) according to claim 6 or 7,
**characterized in that**
the first coil structure (12A) and the second coil structure (12B) are arranged in a common flexible printed circuit board (11), which has a first shaft runout (13A) between the two coil structures (12A, 12B), which deflects the common flexible printed circuit board (11) and forms a first spring element (14A).

9. Fragrancing device (1) according to claim 4 and 6,
**characterized in that**
a magnetic force generated by the energization of the first coil structure (12A) and by the permanent magnet, which moves the first layer (11.1) of the at least one flexible printed circuit board (11) with the first coil structure (12A) facing the outlet opening (5) and the cover section against the force of the at least one spring element (14) from the edge of the outlet opening (5) in the direction of the permanent magnet in the second layer (11.2) of the at least one flexible printed circuit board (11), such that the cover section (16) of the first layer (11.1) opens the outlet opening (5).

10. Fragrancing device (1) according to claim 6 or 9,
**characterized in that**
the first coil structure (12A) and the permanent magnet are arranged in a common flexible printed circuit board (11), which has a first shaft runout (13A) between the first coil structure (12A) and the permanent magnet, which deflects the common flexible printed circuit board (11) and forms a first spring element (14A).

11. Fragrancing device (1) according to claim 8 or 10,
**characterized in that**
an end section (11.3) of the common flexible printed circuit board (11) has a second shaft runout (13B), which deflects the common flexible printed circuit board (11) and forms a second spring element (14B), such that the cover section (16) of the first layer (11.1) of the common flexible printed circuit board (11) facing the outlet opening (5) can be pressed evenly against the edge of the outlet opening (5).

12. Fragrancing device (1) according to claim 11,
**characterized in that**
the common flexible printed circuit board (11) has a recess (18) in each case in the region of the first shaft runout (13A) and in the region of the second shaft runout (13B), such that the first spring element (14A) and the second spring element (14B) each have two spring legs (15).

13. Fragrancing device (1) according to any one of claims 2 to 12,
**characterized in that**
a surface of the cover section (16) of the first layer (11.1) of the at least one flexible printed circuit board (11) facing the outlet opening (5) is designed as a sealing surface.

## Revendications

1. Dispositif diffuseur de parfum (1) avec au moins un réservoir de parfum (3), dans lequel le au moins un réservoir de parfum (3) présente une ouverture de sortie (5) qui peut être fermée par un dispositif de fermeture (7), dans lequel le dispositif de fermeture (7) est conçu en tant qu'actionneur à circuit imprimé (10) flexible, dans lequel l'actionneur à circuit imprimé (10) flexible est conçu en deux couches dans au moins une zone, de sorte qu'une section de recouvrement (16) de l'actionneur à circuit imprimé (10) étanchéifie l'ouverture de sortie (5) à l'état non alimenté et la libère à l'état alimenté, **caractérisé en ce que** l'actionneur à circuit imprimé (10) flexible comprend au moins un circuit imprimé (11) flexible avec une première structure de bobine (12A) pouvant être alimentée en courant et une seconde structure de bobine (12B) pouvant être alimentée en courant, ou au moins un circuit imprimé (11) flexible avec une première structure de bobine (12A) pouvant être alimentée en courant et avec un aimant permanent.

2. Dispositif diffuseur de parfum (1) selon la revendication 1,
**caractérisé en ce que**
à l'état monté de l'actionneur à circuit imprimé (10) flexible, la première structure de bobine (12A) et la section de recouvrement (16) sont disposées dans une première couche (11.1) de l'au moins un circuit imprimé (11) flexible qui est tournée vers l'ouverture de sortie (5) du réservoir de parfum (3), et la seconde structure de bobine (12B) est disposée dans une seconde couche (11.2) de l'au moins un circuit imprimé (11) flexible qui est opposée à l'ouverture de sortie (5) du réservoir de parfum (3).

3. Dispositif diffuseur de parfum (1) selon la revendication 2,
**caractérisé en ce que**
les deux couches (11.1, 11.2) de l'au moins un circuit imprimé (11) flexible sont orientées l'une par rapport à l'autre de sorte que les deux structures de bobine (12A, 12B) soient disposées l'une au-dessus de l'autre.

4. Dispositif diffuseur de parfum (1) selon la revendication 1,
**caractérisé en ce que**
à l'état monté de l'actionneur à circuit imprimé (10) flexible, la première structure de bobine (12A) et la section de recouvrement (16) sont disposées dans une première couche (11.1) de l'au moins un circuit imprimé (11) flexible qui est tournée vers l'ouverture de sortie (5) du réservoir de parfum (3), et l'aimant permanent est disposé dans une seconde couche (11.2) de l'au moins un circuit imprimé (11) flexible qui est opposée à l'ouverture de sortie (5) du réservoir de parfum (3).

5. Dispositif diffuseur de parfum (1) selon la revendication 4,
**caractérisé en ce que**
les deux couches (11.1, 11.2) de l'au moins un circuit imprimé (11) flexible sont orientées l'une par rapport à l'autre de sorte que la première structure de bobine (12A) et l'aimant permanent sont disposés l'un au-dessus de l'autre.

6. Dispositif diffuseur de parfum (1) selon l'une quelconque des revendications 2 à 5,
**caractérisé en ce que**
au moins un élément à ressort (14) presse au moins la section de recouvrement (16) de la première couche (11.1) de l'au moins un circuit imprimé (11) flexible avec la première structure de bobine (12A) de manière étanche contre un bord de l'ouverture de sortie (5), de sorte que les deux couches (11.1, 11.2) de l'au moins un circuit imprimé (11) flexible présentent un écart prédéfini l'une par rapport à l'autre à l'état non alimenté.

7. Dispositif diffuseur de parfum (1) selon les revendications 2 et 6,
**caractérisé en ce que**
une force magnétique générée par l'alimentation des deux structures de bobine (12A, 12B) déplace la première couche (11.1) de l'au moins un circuit imprimé (11) flexible tournée vers l'ouverture de sortie (5) avec la première structure de bobine (12A) et la section de recouvrement (16) contre la force de l'au moins un élément à ressort (14) depuis le bord de l'ouverture de sortie (5) en direction de la seconde structure de bobine (12B) dans la seconde couche (11.2) de l'au moins un circuit imprimé (11) flexible, de sorte que la section de recouvrement (16) de la première couche (11.1) libère l'ouverture de sortie (5).

8. Dispositif diffuseur de parfum (1) selon la revendication 6 ou 7,
**caractérisé en ce que**
la première structure de bobine (12A) et la seconde structure de bobine (12B) sont disposées dans un circuit imprimé (11) flexible commun qui présente, entre les deux structures de bobine (12A, 12B), un faux-rond d'arbre (13A) qui dévie le circuit imprimé (11) flexible commun et forme un premier élément à ressort (14A).

9. Dispositif diffuseur de parfum (1) selon la revendication 4 et 6,
**caractérisé en ce que**
une force magnétique générée par l'alimentation de la première structure de bobine (12A) et par l'aimant permanent déplace la première couche (11.1) de l'au moins un circuit imprimé (11) flexible tournée vers l'ouverture de sortie (5) avec la première structure de bobine (12A) et la section de recouvrement contre la force de l'au moins un élément à ressort (14) depuis le bord de l'ouverture de sortie (5) en direction de l'aimant permanent dans la seconde couche (11.2) de l'au moins un circuit imprimé (11) flexible, de sorte que la section de recouvrement (16) de la première couche (11.1) libère l'ouverture de sortie (5).

10. Dispositif diffuseur de parfum (1) selon la revendication 6 ou 9,
**caractérisé en ce que**
la première structure de bobine (12A) et l'aimant permanent sont disposés dans un circuit imprimé (11) flexible commun qui présente, entre la première structure de bobine (12A) et l'aimant permanent, un faux-rond d'arbre (13A) qui dévie le circuit imprimé (11) flexible commun et forme un premier élément à ressort (14A).

11. Dispositif diffuseur de parfum (1) selon la revendication 8 ou 10,
**caractérisé en ce que**
une section d'extrémité (11.3) du circuit imprimé (11) flexible commun présente un second faux-rond d'arbre (13B) qui dévie le circuit imprimé (11) flexible commun et forme un second élément à ressort (14B), de sorte que la section de recouvrement (16) de la première couche (11.1) du circuit imprimé (11) flexible commun tournée vers l'ouverture de sortie (5) puisse être pressée uniformément contre le bord de l'ouverture de sortie (5).

12. Dispositif diffuseur de parfum (1) selon la revendication 11,
**caractérisé en ce que**
le circuit imprimé (11) flexible commun présente respectivement un évidement (18) dans la zone du faux-rond d'arbre (13A) et dans la zone du second faux-rond d'arbre (13B), de sorte que le premier élément à ressort (14A) et le second élément à ressort (14B) présentent respectivement deux branches à ressort (15).

13. Dispositif diffuseur de parfum (1) selon l'une quelconque des revendications 2 à 12,
**caractérisé en ce que**
l'une des surfaces de la section de recouvrement (16) de la première couche (11.1) de l'au moins un circuit imprimé (11) flexible tournée vers l'ouverture de sortie (5) est conçue en tant que surface d'étanchéité.
